(19) Europäisches Patentamt
European Patent Office
Office européen des brevets

(11) **EP 4 491 107 A1**

(12) **EUROPEAN PATENT APPLICATION**
published in accordance with Art. 153(4) EPC

(43) Date of publication:
**15.01.2025 Bulletin 2025/03**

(21) Application number: **23766650.8**

(22) Date of filing: **01.03.2023**

(51) International Patent Classification (IPC):
**A61B 5/0245** (2006.01)  **A61B 5/0533** (2021.01)

(52) Cooperative Patent Classification (CPC):
**A61B 5/0245; A61B 5/0533**

(86) International application number:
**PCT/JP2023/007447**

(87) International publication number:
**WO 2023/171472 (14.09.2023 Gazette 2023/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.03.2022  JP 2022035751**

(71) Applicant: **National Institute for Materials Science
Tsukuba-shi, Ibaraki 305-0047 (JP)**

(72) Inventors:
• **KAWAKITA, Jin
  Tsukuba-shi, Ibaraki 305-0047 (JP)**
• **KUROSAWA, Takeshi
  Tokyo 152-0003 (JP)**

(74) Representative: **Dehns
10 Old Bailey
London EC4M 7NG (GB)**

(54) **HEART-RATE VARIABILITY MEASUREMENT SYSTEM AND HEART-RATE VARIABILITY MEASUREMENT METHOD**

(57)    The present invention provides a system for easily measuring heart rate variability with a small measurement device. A heart rate variability measurement system of the present invention including: a perspiration detection unit; and a signal arithmetic processing unit, in which the perspiration detection unit includes: a microdroplet detection unit in which a first thin wire and a second thin wire are disposed in juxtaposition with each other on an insulating substrate; and a perspiration signal output unit that measures a change in an electrical characteristic between the first thin wire and the second thin wire and outputs the change as a perspiration signal, the signal arithmetic processing unit includes: a calculation unit; an information storage unit; and a heart rate variation value output unit, while setting time t, a time lag $\Delta t$, a value CH(t) of the perspiration signal at the time t, a heart rate factor $Y(t+\Delta t)$ indicating heart rate variability at time $t+\Delta t$, coefficients a and b, and a predetermined constant as $\alpha$, the calculation unit calculates $Y(t+\Delta t)=a \cdot X(t)+b$ and $X(t)=CH(t)-CH(t-\alpha)$, the information storage unit stores the coefficient a, the coefficient b, and the constant $\alpha$, and the heart rate variation value output unit outputs the heart rate factor Y.

Fig. 2

**Description**

Technical Field

The present invention relates to a heart rate variability measurement system and a heart rate variability measurement method.

Background Art

Circulatory system diseases including heart diseases are serious diseases that are high-ranked diseases in the number of deaths in Japan, and are dangerous diseases that may suddenly attack without noticing abnormalities. Therefore, monitoring of a circulatory system including a heart is very important for building a healthy society.

In addition, since the frequency characteristic of a temporal change in intervals between heartbeats is considered to correlate with autonomic nerve balance, heart rate variability measurement can be effectively utilized for management of a mental state and a biological rhythm.

One of important monitoring indicators of a circulatory system is heart rate variability.

Although the heart rate variability can be monitored by an electrocardiogram or the like, the electrocardiogram is a relatively large-scale device used in a medical institution or the like, and it is difficult to use the electrocardiogram for monitoring easily anytime and anywhere.

In response to such a situation, a compact and portable device has been developed, and is disclosed in, for example, Patent Literature 1.

It is described in Patent Literature 1 a method of detecting acceleration of a substrate attached to clothing with a sensor and deriving a heart rate from the acceleration. In this method, two sensors are used so that abnormality measurement data associated with posture disorders or the like is not captured, but there is a problem that it is difficult to always perform highly reliable heart rate variability measurement because only vibration is measured.

For this reason, there is a strong demand for a heart rate variability measurement system capable of easily measuring heart rate variability at a body end portion such as a finger with a small device.

Citation List

Patent Literature

Patent Literature 1: JP 2002-345769 A
Patent Literature 2: WO 2016/013544 A

Summary of Invention

Technical Problem

An object of the present invention is to provide a system for easily measuring heart rate variability at a hand portion such as a finger with a small measurement device, and a measurement method thereof.

Solution to Problem

A configuration of the present invention for solving the above problems will be described below.

(Configuration 1)

A heart rate variability measurement system comprising:

a perspiration detection unit, and a signal arithmetic processing unit, wherein
the perspiration detection unit includes a microdroplet detection unit in which a first thin wire and a second thin wire are disposed in juxtaposition with each other on an insulating substrate, and a perspiration signal output unit that measures a change in an electrical characteristic between the first thin wire and the second thin wire and outputs the change as a perspiration signal,
the signal arithmetic processing unit includes a calculation unit, an information storage unit, and a heart rate variation value output unit,

while setting time t, a time lag $\Delta t$, a value CH(t) of the perspiration signal at the time t, a heart rate factor Y(t+$\Delta t$) indicating heart rate variability at time t+$\Delta t$, coefficients a and b, and a predetermined constant as $\alpha$, the calculation unit calculates following equations (1) and (2):

$$Y(t+\Delta t)=a \cdot X(t)+b \quad \cdots (1)$$

$$X(t)=CH(t)-CH(t-\alpha) \quad \cdots (2),$$

the information storage unit stores at least the coefficient a, the coefficient b, and the constant $\alpha$, and
the heart rate variation value output unit outputs the heart rate factor Y,
where the coefficient a and the coefficient b are determined by obtaining a heart rate measurement heart rate factor Y' that is calculated from measurement of a heart rate value HB(t) at the time t, performed in advance simultaneously with the measurement of the perspiration signal by the heart rate variability measurement system, according to an equation (3):

$$Y'(t+\Delta t)=HB(t+\Delta t)-HB(t+\Delta t-\alpha) \quad \cdots (3)$$

and performing regression analysis of an equation (4):

$$Y'(t+\Delta t)=a \cdot X(t)+b \quad \cdots (4).$$

(Configuration 2)

The system as described in Configuration 1, wherein the perspiration detection unit includes a microdroplet detection unit in which a thin wire of first metal and a thin wire of second metal, are disposed in juxtaposition with each other on an insulating substrate, the second metal being different from the first metal, and a perspiration signal output unit which measures a galvanic current flowing between the thin wire of the first metal and the thin wire of the second metal and outputs the galvanic current as a perspiration signal.

(Configuration 3)

The system as described in Configuration 2, wherein the first metal is selected from a group consisting of gold, platinum, silver, titanium, an alloy thereof, and carbon.

(Configuration 4)

The system as described in Configuration 2 or 3, wherein the second metal is selected from a group consisting of silver, copper, iron, zinc, nickel, cobalt, aluminum, tin, chromium, molybdenum, manganese, magnesium, and an alloy thereof.

(Configuration 5)

The system as described in any one of Configurations 1 to 4, wherein the constant $\alpha$ is 0.2.

(Configuration 6)

The system as described in any one of Configurations 2 to 5, comprising a plurality of at least one selected from the group consisting of the thin wire of the first metal and the thin wire of the second metal, wherein the thin wire of the first metal extends from a first side towards a second side that is opposite to the first side and the thin wire of the second metal extends from the second side towards the first side such that the thin wire of the first metal and the thin wire of the second metal run in parallel.

(Configuration 7)

The system as described in any one of Configurations 2 to 6, wherein a spacing between the thin wire of the first metal and the thin wire of the second metal is 0.1 $\mu$m or more and 10 $\mu$m or less.

(Configuration 8)

The system as described in any one of Configurations 1 to 7, wherein the perspiration detection unit and the signal arithmetic processing unit are placed at physically distant places.

(Configuration 9)

The system as described in Configuration 8, wherein perspiration signal data output from the perspiration detection unit is transmitted to the signal arithmetic processing unit by communication.

(Configuration 10)

The system as described in Configuration 8, wherein perspiration signal data output from the perspiration detection unit is transmitted to the signal arithmetic processing unit via an electronic medium.

(Configuration 11)

A heart rate variability measurement method comprising:

acquiring a value CH(t) of a perspiration signal at time t using a perspiration detection device that includes a microdroplet detection unit in which a thin wire of first metal and a thin wire of second metal are disposed in juxtaposition with each other on an insulating substrate, the second metal being different from the first metal, and a perspiration signal output unit which measures a galvanic current flowing between the thin wire of the first metal and the thin wire of the second metal and outputs the galvanic current as a perspiration signal,
while setting a time lag $\Delta t$, a heart rate factor $Y(t+\Delta t)$ indicating heart rate variability at time $t+\Delta t$, coefficients a and b, and a predetermined constant as $\alpha$, calculating following equations (1) and (2):

$$Y(t+\Delta t)=a\cdot X(t)+b \quad \cdots (1)$$

$$X(t)=CH(t)-CH(t-\alpha) \quad \cdots (2),$$

and

outputting the heart rate factor Y thus calculated,
where the coefficient a and the coefficient b are determined by obtaining a heart rate measurement heart rate factor Y' that is calculated from measurement of a heart rate value HB(t) at the time t, performed in advance simultaneously with the measurement of the perspiration signal by the perspiration detection device, as described in an equation (3):

$$Y'(t+\Delta t)=HB(t+\Delta t)-HB(t+\Delta t-\alpha) \quad \cdots (3)$$

and performing regression analysis of an equation (4):

$$Y'(t+\Delta t)=a\cdot X(t)+b \quad \cdots (4).$$

(Configuration 12)

The heart rate variability measurement method as described in Configuration 11, wherein the constant $\alpha$ is 0.2.

Advantageous Effects of Invention

The present invention provides a system for easily measuring heart rate variability at a hand portion such as a finger with a small measurement device, and a measurement method thereof.

Brief Description of Drawings

Fig. 1 shows an explanatory diagram indicating a configuration of a heart rate variability measurement system of the present invention.
Fig. 2 shows an explanatory diagram indicating the configuration of the heart rate variability measurement system of the present invention.
Fig. 3 shows an explanatory diagram indicating a configuration of a microdroplet detection unit of the system of the present invention and an operation principle thereof.
Fig. 4 shows a sectional view indicating a structure of a main part of the microdroplet detection unit of the system of the present invention.
Fig. 5 shows a sectional view indicating another structure of the main part of the microdroplet detection unit of the system of the present invention.
Fig. 6 shows a sectional view indicating a structure of a main part of a specimen unit of the system of the present invention.
Fig. 7 shows an explanatory diagram indicating a configuration of a signal arithmetic processing unit of the system of the present invention.
Fig. 8 shows a characteristic diagram indicating an example of measurement signals of an electrocardiogram.
Fig. 9 shows a characteristic diagram indicating an example of perspiration signals in the present invention.
Fig. 10 shows a photograph indicating an appearance of a perspiration sensor used in an example.
Fig. 11 shows an optical photograph indicating the microdroplet detection unit of the perspiration sensor used in the example taken from an upper surface.
Fig. 12 shows an explanatory diagram indicating a measurement procedure in an example.
Fig. 13 shows a characteristic diagram indicating a correlation between perspiration signal data and heart rate value data.
Fig. 14 shows a characteristic diagram indicating the correlation between the perspiration signal data and the heart rate value data.

Description of Embodiments

Hereinafter, embodiments of the present invention will be described with reference to the drawings.
First, a heart rate variability measurement system of the present invention will be described.

<System Overview>

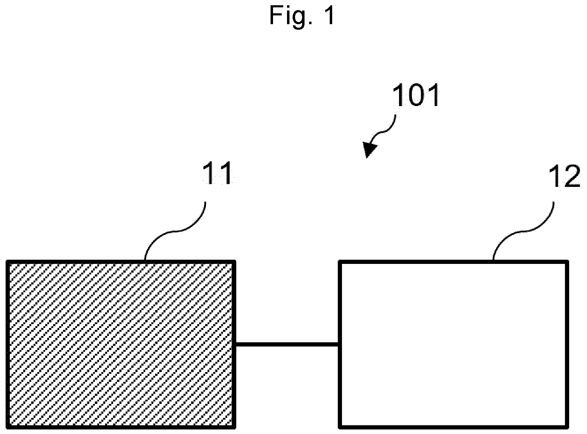

As shown in Fig. 1, a heart rate variability measurement system 101 of the present invention includes a perspiration detection unit 11 and a signal arithmetic processing unit 12.

<Perspiration Detection Unit>

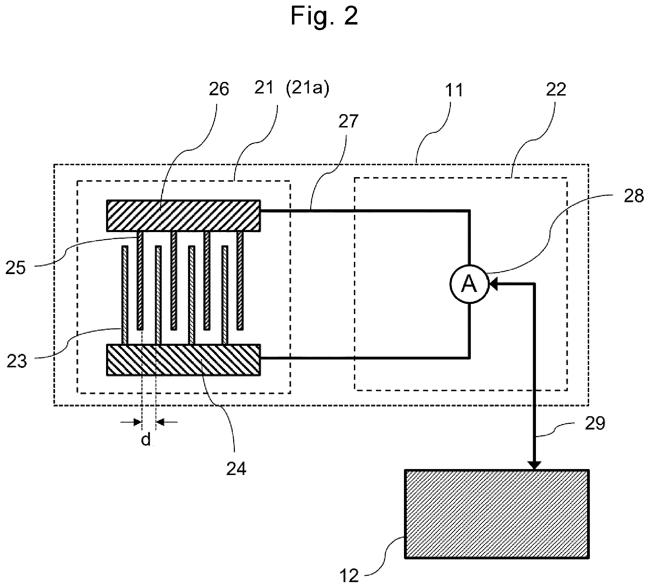

The perspiration detection unit 11 is a means that measures the transpiration rate (perspiration rate) of transpiration water caused by perspiration from a hand of a subject, and includes a specimen unit 21 and a measurement unit 22 as shown in Fig. 2 which is a plan view.
The specimen unit 21 includes a case that houses at least a part of the hand of the subject and a microdroplet detection unit 21a that detects a microdroplet having a size of 100 nm or more and 20 $\mu$m or less in terms of diameter.
In this context, the hand means parts including a finger, a palm, and a wrist, and the perspiration detection unit 11 measures the transpiration rate of transpiration water associated with perspiration from any one or more of the parts. Therefore, the transpiration rate of transpiration water may be measured from any of the finger alone, the palm alone, the wrist alone, the finger and the palm, the palm and the wrist, and the finger, the palm, and the wrist.
The measurement at the finger (more specifically, a fingertip) has a feature that it is easy to reduce the size and weight of the device to be used (perspiration detection device), it is simple, excellent in handleability, and it is easy to reduce the cost.

The measurement at the palm has a feature that the perspiration rate can be accurately and easily measured since the amount of perspiration from this part is large.

The measurement at the wrist, which is close to a trunk, makes it possible to perform measurement with less influence of other factors other than perspiration.

Naturally, the measurement using multiple parts such as the finger and the palm makes it possible to perform measurement having the features of the individual parts.

The case may accommodate the part of the hand to be measured. Alternatively, the case may have such a structure that perspiration from a part of the hand to be measured is less likely to be released when the case is brought into close contact with or close proximity to the part of the hand to be measured. For example, the case may have a box shape in which a hand insertion port is formed or a cup shape in which a measurement part such as a finger can be placed instead of a lid.

In addition, the case can be configured to have a small cavity (space capacity) to a degree that the hand to be measured does not touch the microdroplet detection unit 21a at the time of measurement so that the perspiration rate can be measured in a short time.

Therefore, the volume of the specimen unit, which is a space formed in the case at the time of measurement, is preferably 0.05 cm$^3$ or more and 2.5 cm$^3$ or less.

The microdroplet detection unit 21a detects transpiration water caused by perspiration as microdroplets. As a configuration of the microdroplet detection unit 21a, a configuration of a sensor unit of a droplet sensor that monitors electric resistance between thin wires, a change in capacitance, a galvanic current flowing between the thin wires, and the like can be adopted. Among such droplet sensors, a type of them that detects transpiration water associated with perspiration in a state of droplets or aggregated water molecules is preferable, in terms of high responsiveness to a target to be detected, as compared with a type of them that absorbs transpiration water using a porous layer or the like.

Among them, from the viewpoint of responsiveness, a galvanic current measuring system that includes a thin wire(s) of first metal (first thin metal wire(s)) 23 and a thin wire(s) of second metal (second thin metal wire(s)) 25, which are disposed in juxtaposition with each other on an insulating substrate at a small spacing d, the second metal being different from the first metal, and measures a galvanic current flowing between the first thin metal wire(s) 23 and the second thin metal wire(s) 25 is particularly preferable. In such a system, the first thin metal wire(s) 23 can be electrically connected and bundled to a first electrode 24 as a collector electrode, and the second thin metal wire(s) 25 can be electrically connected and bundled to a second electrode 26 as a collector electrode.

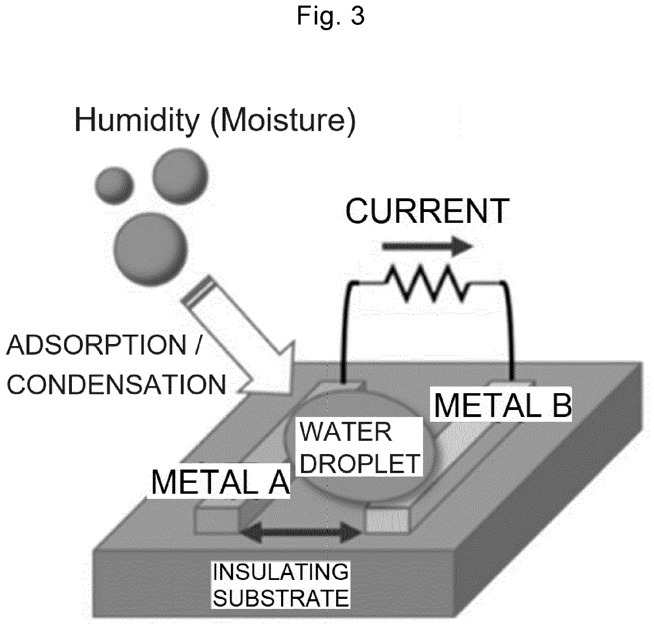

Fig. 3 shows a principle by which microdroplets can be detected by the microdroplet detection unit 21a employing the galvanic current measuring system. It is noted that, this principle is also described in Patent Literature 2.

Moisture in transpiration water drifts in the air as minute droplets, droplets (water droplets containing impurity ions from sweat) are formed so as to bridge the first thin metal wire(s) 23 made of metal A and the second thin metal wire(s) 25 made of metal B by an adsorption/condensation phenomenon, and a galvanic current flows due to an electrochemical potential difference between these kinds of metal. Since the magnitude of this galvanic current is correlated with the number and size of droplets, a change rate of the galvanic current corresponds to a microdroplet formation rate, that is, a perspiration rate. This correlation can be quantitatively understood if a calibration curve is obtained in advance. Therefore, the perspiration rate can be obtained by monitoring the change rate of the galvanic current.

As the change rate of the galvanic current, the first derivative of a galvanic output current, the reciprocal of the rise time until the galvanic output current value set based on certain criteria is reached, or the like can be used.

The above-mentioned system utilizes a physical adsorption detecting method as a perspiration rate measuring method, and it is different from a chemical adsorption detecting method used in a typical humidity sensor.

The physical adsorption detecting method, that is, the method in which moisture caused by perspiration is detected in the microdroplet detection unit 21a, including the first thin metal wires 23 and the second thin metal wires 25 disposed in juxtaposition with each other on the insulating substrate at the small spacing d, has a feature in its accurate responsiveness according to the amount of moisture even under high humidity since droplets can be adsorbed on a sensor surface (microdroplet detection surface) in a stacked manner. On the other hand, in the chemical adsorption detecting method, since the adsorption capacity is limited due to monolayer adsorption, when the amount of perspiration, that is, the amount of droplets due to perspiration increases, adsorption saturation occurs and measurement accuracy decreases.

As shown in Fig. 2, in the case where a plurality of at least one selected from the group consisting of the first thin metal wire 23 and the second thin metal wire 25 are comprised, the first thin metal wire 23 extends from a first side towards a second side that is opposite to the first side and the second thin metal wire 25 extends from the second side towards the first side such that the first thin metal wire 23 and the second thin metal wire 25 run in parallel, and these wires are formed of an electrode having a narrow electrode width, that is, a thin wire, it is possible to increase a length of a portion where both electrodes are faces each other with approaching each other while suppressing the area occupied by the microdroplet detection unit 21a. This can increase a capacity of the cell, that is, increase a galvanic current that can be output. The increase in the galvanic current is preferable because S/N in perspiration rate measurement is improved.

As a configuration for increasing a length (hereinafter, referred to as an approaching distance) of approached portions between thin wire electrodes by arranging such thin wire electrodes in parallel with and approached each other, for example, a comb structure or a double spirally-wound structure may be employed. In addition, a structure itself for increasing an approaching distance between two wirings inside a predetermined plane area as possibly as can be is well known in the field of a semiconductor device and the like, and thus, such a structure may be employed as is necessary. In the present invention, "juxtaposing thin wires (thin wire electrodes) on a substrate" is not for specifying mutual directions of a plurality of thin wire electrodes placed on the substrate but represents that the thin wires on a same plane of the substrate with being separate from each other.

In a case where the first thin metal wire 23 is used as a cathode, examples of the material of the first thin metal wire 23 include a material selected from a group consisting of gold (Au), platinum (Pt), silver (Ag), titanium (Ti), an alloy thereof, and carbon (C).

In a case where the second thin metal wire 25 is used as an anode, examples of the material of the second thin metal wire 25 include a material selected from the group of silver (Ag), copper (Cu), iron (Fe), zinc (Zn), nickel (Ni), cobalt (Co), aluminum (Al), tin (Sn), chromium (Cr), molybdenum (Mo), manganese (Mn), magnesium (Mg), and an alloy thereof. It is noted that, in a case where silver or an alloy thereof are used as the first thin metal wire 23, a material other than silver and an alloy thereof is used as the material for the second thin metal wire 25.

The output (current) depends on the combination of materials of the thin metal wires. For example, when the combinations of silver/iron and gold/silver are compared with each other, the current value to be obtained in the combination of silver/iron is larger than that in the combination of gold/silver because the combination of silver/iron has a corrosion rate per area larger than that in the combination of gold/silver. On the other hand, in the combination of gold/silver, the service life is longer because the combination of gold/silver has smaller consumption of the electrodes. In this regard, silver has an effect of preventing the generation of mold at a place where a water droplet is detected, and therefore, it is preferable to use silver as the first thin metal wire 23 or the second thin metal wire 25.

Further, in a case where the first electrode 24 is made of the same material as that of the first thin metal wire 23, and the second electrode 26 is made of the same material as that of the second thin metal wire 25, the production process of the perspiration detection device is simplified, and therefore, this is preferable.

The spacing d between the first thin metal wire 23 and the second thin metal wire 25 is preferably 0.1 $\mu$m or more and 10 $\mu$m or less, more preferably 1.0 $\mu$m or more and 5 $\mu$m or less, still more preferably 1.5 $\mu$m or more and 3 $\mu$m or less. The inventors have found, through the accumulation of a large amount of experimental data, that high resolution and high measurement reproducibility of the perspiration rate can be obtained when the spacing d is in this range.

The first thin metal wire 23 and the second thin metal wire 25 preferably have a thickness of 10 nm or more and 300 nm or less. When the the first thin metal wire 23 and the second thin metal wire 25 have a thickness less than 10 nm, electrical resistance becomes too large to produce an output and tends to cause a large time-dependent change of the output. On the other hand, even when the first thin metal wire 23 and the second thin metal wire 25 have a thickness over 300 nm, no particular effect is observed, resulting in waste of material.

It is noted that, when a galvanic current flows between the first thin metal wire 23 and the second thin metal wire 25 repeatedly, the metal of the thin metal wire on the anode side is ionized, and accordingly, the thin metal wire on the anode side is gradually consumed. In particular, when the thin metal wire is thinned for increasing the laying density, there is a possibility that the inter-thin wire electrode distance is gradually increased or the thin wire electrode is cut out in accordance with the consumption of the thin metal wire on the anode side.

In order to address these problems with the laying density of the thin metal wires maintained, for example, the thickness of the thin metal wire on the anode side may be increased, or the width of the thin metal wire on the anode side may be increased instead of the width of the thin metal wire on the cathode side may be decreased.

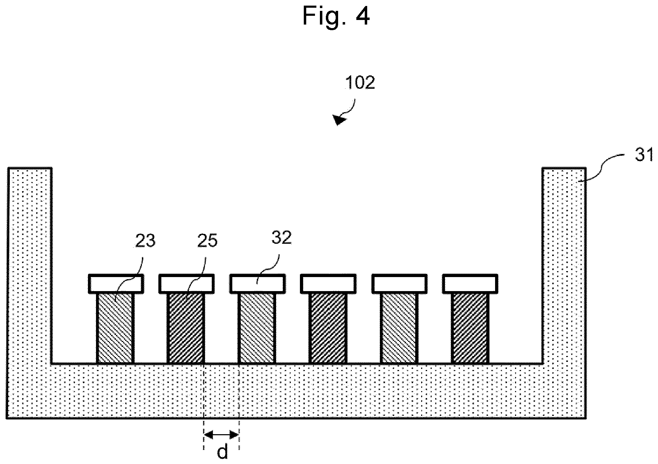

If the first thin metal wire 23 and the second thin metal wire 25 come into contact with the hand or sweat that drips directly from the hand, the measurement accuracy of the perspiration rate decreases. Therefore, as shown in Fig. 4 describing the structure of a specimen unit 102, it is preferable to form protective caps 32 on the upper surfaces of the first thin metal wire 23 and the second thin metal wire 25 arranged in a case 31. In order to enhance the protection performance against unnecessary sweat, the protective cap 32 preferably has a so-called overhang shape having an eave for the first thin metal wire 23 and the second thin metal wire 25. Meanwhile, when a part of the hand to be measured is placed inside the case, or when the part of the hand to be measured and the case are brought into close contact with or close proximity to each other, internal static electricity is discharged from the hand (for example, fingertip) by the hand touching or approaching the protective cap 32, which may cause an unintended current value to be measured. Therefore, it is also preferable to make a part or all of the material of the protective cap 32 conductive so that internal static electricity can be released when the hand touches or approaches the protective cap 32. However, in this case, the protective cap 32 has such a configuration as to be electrically insulated from the first thin metal wire 23 and the second thin metal wire 25.

Examples of the film used for the protective cap 32 include a single-layer film selected from a group consisting of $SiO_2$, SiON, $SiO_x$, $SiN_x$, $Si_3N_4$, $HfO_x$, $Al_2O_3$, polyimide, acrylic, polystyrene (PS), polypropylene (PP), polyethylene terephthalate (PET), polycarbonate (PC), polytetrafluoroethylene (PTFE), tetrafluoroethylene-perfluoroalkyl vinyl ether copolymer (PFA), tetrafluoroethylene-hexafluoropropylene copolymer (FEP), tetrafluoroethylene-ethylene copolymer (ETFE), and copper, aluminum, nickel, zinc, and an alloy thereof, and stainless steel, or a laminated film including one or more selected from the group.

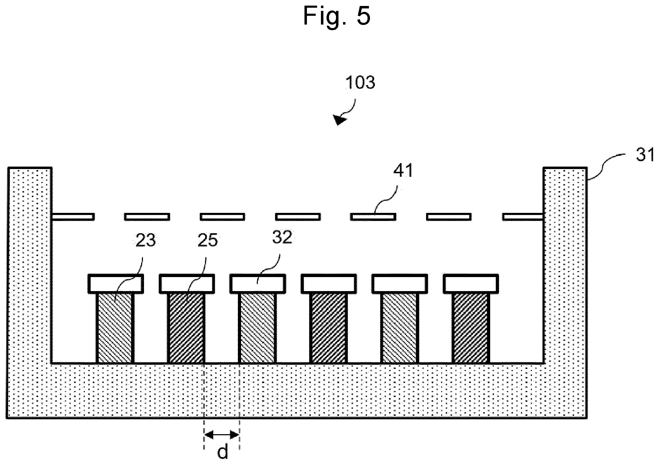

In addition, as shown in Fig. 5 describing the structure of another specimen unit 103, it is preferable to provide a protective mesh 41 in which an opening, through which gas permeates, is formed above the first thin metal wire 23, the second thin metal wire 25, and the protective cap 32. The opening of the protective mesh 41 enables moisture (moisture of transpiration water) caused by perspiration to pass through the protective mesh 41. On the other hand, since the protective mesh 41 is provided, it is possible to prevent the hand from touching the first thin metal wire 23 and the second thin metal wire 25.

Examples of the protective mesh 41 include, but are not limited to, a mesh member such as a mesh plate or a mesh film, a woven fabric, a nonwoven fabric, and the like. The material of the protective mesh 41 is not particularly limited, and metal, oxide, nitride, oxynitride, silicon, organic substance, or the like can be used. The opening of the protective mesh 41 can be achieved by a hole or groove formed in the protective mesh material, a space portion of a fiber observed when a cloth is used for the protective mesh material, or the like which can ensure a path allowing penetration of moisture.

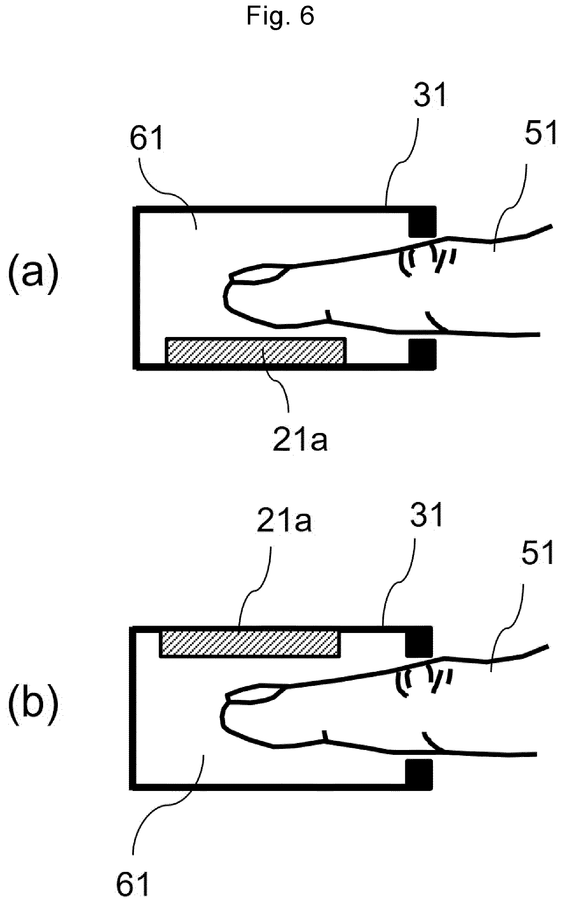

In addition, as shown in Fig. 6 (a), the microdroplet detection unit 21a in which the first thin metal wire 23 and the second thin metal wire 25 are arranged may be arranged below the hand (a finger 51 in the case of Fig. 6 (a)), or may be arranged above (Fig. 6 (b)) or laterally while keeping an interval so as not to come into contact with the hand. When the microdroplet detection unit 21a is arranged above, it is possible to prevent sweat that drips directly from the hand from touching the first thin metal wire 23 and the second thin metal wire 25 while making a space 61 (capacity of the specimen unit) compact, so that measurement accuracy and measurement reliability of the perspiration rate are improved. It is noted that, as described above, the space 61 is preferably as small as possible in order to measure the perspiration rate for a short time.

The measurement unit 22 includes an ammeter 28 electrically connected to the first metal electrode 24 and the second metal electrode 26 via wiring 27, and can measure a galvanic current generated by the first thin metal wire 23 and the second thin metal wire 25. Then, the current value and the temporal change of the current value are sent to the signal arithmetic processing unit 12 by a signal path 29 as a perspiration signal. In other words, the measurement unit 22 functions as a perspiration signal output unit that measures a change in the galvanic current between the first thin metal wire 23 and the second thin metal wire 25 and outputs the change as a perspiration signal. The signal path 29 may be electric wiring, a wireless distribution, or an electronic medium, but is preferably electric wiring or a wireless distribution from the viewpoint of quick response.

The measurement unit (perspiration signal output unit) 22 may be placed inside, outside, or across the inside and outside of the case 31 constituting the specimen unit 21. When the measurement unit is placed inside the case, the perspiration detection device can be made compact. When the measurement unit is placed outside the case, the following effects can be obtained: (1) maintainability is improved; (2) a failure rate is reduced because the measurement unit (perspiration signal output unit) 22 can be placed in an environment relatively lower in humidity than the inside of the case; and (3) measurement accuracy and measurement reliability of a perspiration rate are improved because the influence of heat generation from the device hardly affects the inside of the case. In addition, when the measurement unit is placed across the inside and outside of the case, a moderate effect can be obtained in the case of being placed inside the case and the case of being placed outside the case.

<Signal Arithmetic Processing Unit>

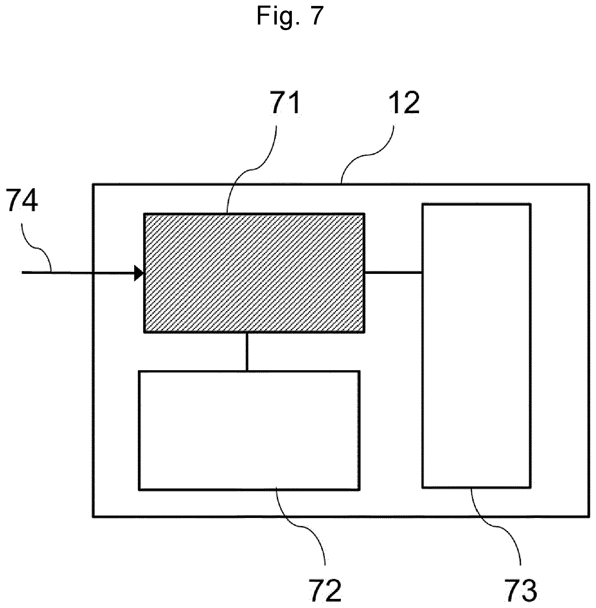

As shown in Fig. 7, the signal arithmetic processing unit 12 includes a calculation unit 71, an information storage unit 72, and a heart rate variation value output unit 73.

The calculation unit 71 sets time t, a time lag $\Delta t$, a value CH(t) of a perspiration signal 74 (input signal) at the time t, a heart rate factor Y(t+$\Delta t$) indicating heart rate variability at time t+$\Delta t$, coefficients a and b, and a predetermined constant as $\alpha$, and calculates the following equations (1) and (2).

$$(t+\Delta t)=a \cdot X(t)+b \quad \cdots (1)$$

$$X(t)=CH(t)-CH(t-\alpha) \quad \cdots (2)$$

The information storage unit 72 stores at least the coefficients a and b and the constant $\alpha$.

The heart rate variation value output unit 73 outputs a heart rate factor Y.

The coefficients a and b are determined by obtaining a heart rate measurement heart rate factor Y' that is calculated from measurement of a heart rate value HB(t) at the time t, performed in advance simultaneously with the perspiration signal measurement by the heart rate variability measurement system 101, according to the equation (3):

$$Y'(t+\Delta t)=HB(t+\Delta t)-HB(t+\Delta t-\alpha) \quad \cdots (3)$$

and performing regression analysis of the equation (4):

$$Y'(t+\Delta t)=a \cdot X(t)+b \quad \cdots (4).$$

Typically, the constant $\alpha$ is preferably 0.2. It has been found from extensive experiments that when the constant $\alpha$ is set to 0.2, a high T value is obtained, the degree of relationship between X and Y increases, and the accuracy of the heart rate variability measurement increases.

As described above, the heart rate measurement heart rate factor Y' calculated by the above equation (3) is a heart rate factor obtained using the constant $\alpha$ on the basis of the measurement of the heart rate value HB(t) at the time t performed in advance, and is different from the heart rate factor Y obtained by the above equation (1) in that it is a factor having a primary relationship with X(t) (that is, the perspiration signal change) obtained by the above equation (2) using the perspiration signal value CH(t) at the time t and the constant $\alpha$. For the purpose of distinguishing both, the former is represented using a symbol "Y'", whereas the latter is represented using a symbol "Y". On the other hand, as understood from comparison between the above equations (1) and (4), the heart rate factor Y and the heart rate measurement heart rate factor Y' are essentially the same factor.

In this context, the equations (3) and (4) used for determining the coefficients a and b can also be arranged as follows by replacing the symbol "Y'" with "Y".

$$Y(t+\Delta t)=HB(t+\Delta t)-HB(t+\Delta t-\alpha)$$

$$X(t)=CH(t)-CH(t-\alpha)$$

$$Y(t+\Delta t)=a \cdot X(t)+b$$

Here, the determination of the coefficients a and b will be exemplarily described.

In determining the coefficients a and b, it is preferable to acquire a large amount of time-series data of the heart rate value HB(t) and the perspiration signal value CH(t). In order to increase the number of data to be acquired, it may be effective not only to increase the number of times of data acquisition (frequency) but also to divide one measurement period (measurement time) into multiple frames and extract data from each frame.

Regarding the latter, for example, in a case where one measurement period is set to 60 seconds, it is considered that 60 seconds are divided into multiple frames with 4 seconds set as one frame. The time range of one frame may be shorter or longer than 4 seconds, and may be set so that beats that can be converted into beat per minutes (bpm), which is usually used as a unit of the heart rate value, can be checked. When the predetermined measurement period is divided into set frames, the measurement period can be divided into more frames by providing as many start points (starting points) of the frames as possible. In the above example, since the measurement period is 60 seconds and one frame is 4 seconds, the start point of the frame is provided in a frame from 0 seconds at the measurement start time point to 56 seconds (time). For example, in a case where the start point is provided every 0.05 seconds, it is possible to obtain (56/0.05 = 1120) frames, and it is possible to acquire time-series data of the heart rate value HB(t) and the perspiration signal value CH(t) from each of these frames.

Furthermore, by changing the time lag $\Delta t$ in a certain range for the time-series data obtained in this manner, it is possible to obtain the number of pieces of data to be analyzed corresponding to the number of set values of the time lag $\Delta t$ from one time-series data.

Regression analysis is performed using the large amount of data to be analyzed thus obtained. Typically, single regression analysis can be performed as a regression analysis method. As a result, a determination coefficient and a correlation coefficient are obtained from each piece of data to be analyzed together with the regression coefficients a and b.

Thereafter, using a T value of the correlation coefficient as an index, the correlation coefficient having a T value exceeding +2 is extracted. This makes it possible to increase the reliability of the values of the determined coefficients a and b.

Then, an average value of the regression coefficients a and b is obtained from the extracted data group, and this value is used as a determined value of the coefficients a and b and stored in the information storage unit 72.

The perspiration detection unit 11 and the signal arithmetic processing unit 12 may be housed in one housing, or may be placed at physically distant places. In the latter case, the perspiration signal 74 measured by the perspiration detection unit 11 may be transmitted from the perspiration detection unit 11 to the signal arithmetic processing unit 12 by communication (wireless communication), may be transmitted via an electronic medium such as a USB, a memory card, or a disk, or may be transmitted by wired connection. Among these, transmission through wireless communication is efficient in that the perspiration detection device attached to the hand can be reduced in size and weight like a finger cot and the signal processing unit 12 can receive data from many subjects and perform arithmetic processing in large amounts simultaneously in parallel. In the case of using wired connection, a wireless facility is unnecessary, and thus it is easier to reduce the size and weight of the system and to suppress the cost. The method using an electronic medium has a feature that time-series data processing for a relatively long period can be efficiently performed.

<Measurement Method>

Next, a heart rate variability measurement method by the system of the present invention will be described.

The heart rate variability measurement method of the present invention measures the value CH(t) of the perspiration signal at the time t using the heart rate variability measurement system (perspiration detection device), calculates the above-described equations (1) and (2) by using the time lag $\Delta t$, the heart rate factor $Y(t+\Delta t)$ indicating heart rate variability at the time $t+\Delta t$, the coefficients a and b, and the predetermined constant as $\alpha$, and outputs the calculated heart rate factor Y.

The coefficients a and b are determined by obtaining the heart rate measurement heart rate factor Y' calculated from the measurement of the heart rate value HB(t) at the time t, performed in advance simultaneously with the perspiration signal measurement by the heart rate variability measurement system (perspiration detection device), according to the equation (3) and performing regression analysis of the equation (4).

Typically, the constant $\alpha$ is preferably 0.2. When the constant $\alpha$ is set to 0.2, a high T value is obtained, the degree of relationship between X and Y increases, and the accuracy of the heart rate variability measurement increases.

The determination of the coefficients a and b is as exemplarily described above.

With this measurement method, heart rate variability can be easily measured with a compact device at a hand portion such as a finger that is easy to handle.

Examples

(Example 1)

The inventors have found from extensive experiments that a perspiration rate (amount of perspiration per unit time) from a body end portion such as a finger is correlated with heart rate variability at a specific time lag. Then, based on the finding, the inventors have invented a method of measuring heart rate variability from the perspiration rate through formulation based on regression analysis. Examples thereof are shown below.

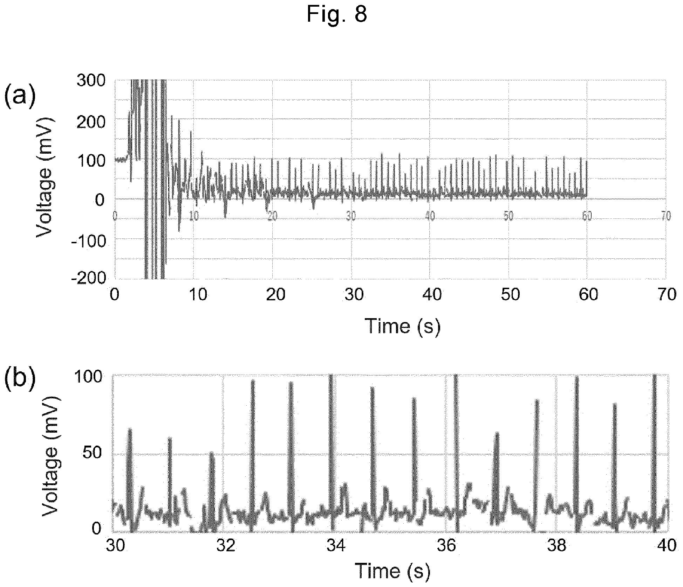

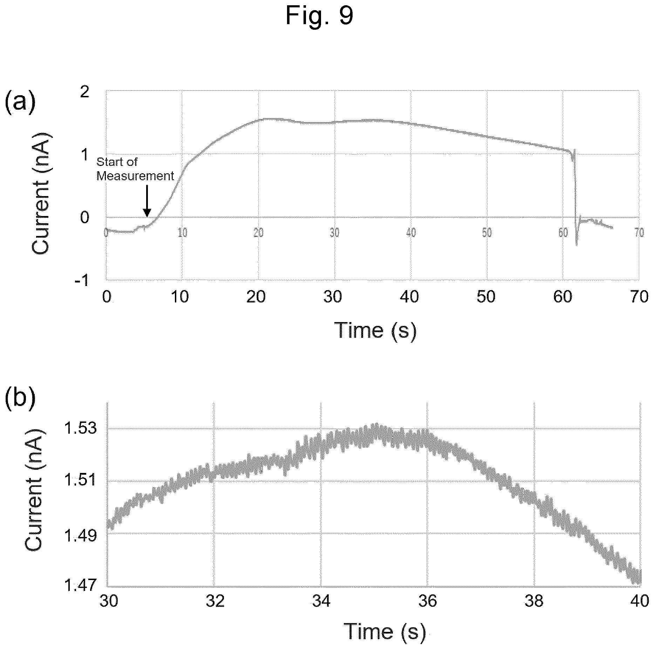

Fig. 8 shows an example of waveform data of an electrocardiogram frequently used in the medical field as a method of monitoring a state of a heart rate, that is, movement of a heart. Fig. 9 shows an example of perspiration signals measured by a perspiration sensor prototyped as a perspiration detection device including the perspiration detection unit 11 of the system of the present invention. In both Figs. 8 and 9, part (a) shows a measurement result in a time frame (time) 0 to 60 s, and part (b) shows a result obtained by magnifying the range of the time frame (time) 30 to 40 s in part (a). In the electrocardiogram measurement shown in Fig. 8, after an electrocardiograph (manufactured by Parama Tech Co., Ltd., EP-501) was activated, data for 60 seconds after a waveform of the electrocardiogram started to display was recorded. In the perspiration signal measurement shown in Fig. 9, with a time point at which recording of the perspiration signal (current value) output from the perspiration sensor was started set to 0 s, a subject performed an operation of placing a finger on the specimen unit of the perspiration sensor at a time of about 5 s (measurement start) and releasing the finger from the specimen unit at a time of about 62 s.

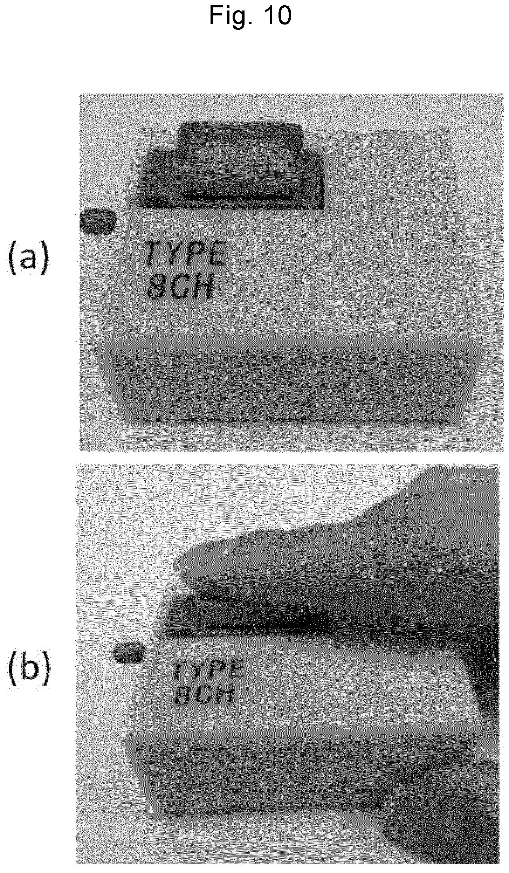

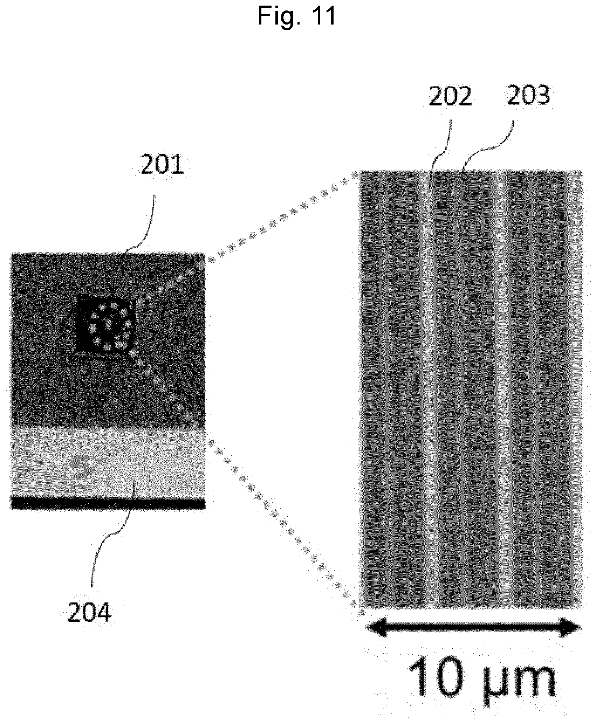

Fig. 10 shows an appearance photograph of the perspiration sensor used in the present embodiment, and Fig. 11 shows an optical photograph obtained by photographing a sensor unit (corresponding to the microdroplet detection unit 21a) from the upper surface.

In Fig. 10, part (a) is a photograph of the perspiration sensor alone, and part (b) is a photograph of a state in which the finger is placed on a case of this sensor. The case of the specimen unit has a cup shape having a substantially rectangular shape in plan view, and has an opening on the upper side. As shown in Fig. 10(b), when the subject places a finger (covers the case with the finger), the finger serves as a lid.

Fig. 11 (a) is an optical photograph of the microdroplet detection unit of the perspiration sensor, in which the left side is an image obtained by photographing a thin metal wire (galvanic array) arranged in a comb shape on a silicon chip (insulating substrate) 201 together with a scale 204, and the right side is an image obtained by magnifying and observing a first thin metal wire 202 and a second thin metal wire 203 from the upper surface.

As shown in Fig. 10(b), the perspiration sensor is configured to be able to measure the rate (perspiration rate) of sweat evaporating from the finger (fingertip) when the finger is placed on the case and transmit measurement data to a server by wireless communication. In the present embodiment, the first thin metal wire 202 is made of aluminum (Al), the second thin metal wire 203 is made of gold (Au), and the spacing between the first thin metal wire 202 and the second thin metal wire 203 was set to 1 μm.

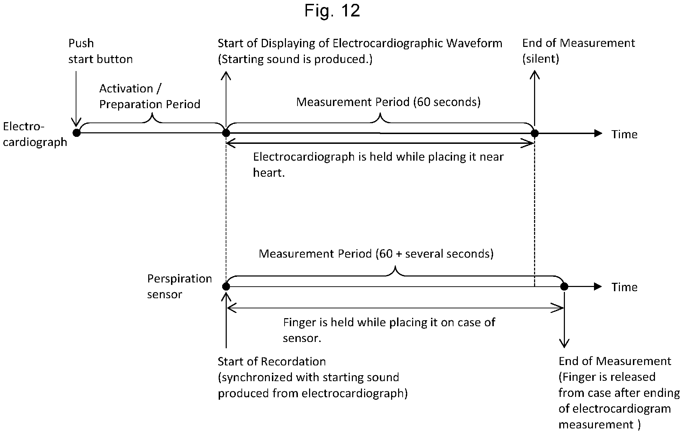

Using such a perspiration sensor and an electrocardiograph, measurement was performed on the subject according to a procedure shown in Fig. 12, and data of the perspiration signal CH(t) and the heart rate value HB(t) were acquired. The number of subjects was a total of 4 persons including 2 females in their 50s and 2 males in their 20s. Briefly describing the measurement procedure, the measurement time of the electrocardiograph was set to 60 seconds, and the perspiration signals output from the perspiration sensor were started to be recorded at the time when the waveform of the electrocardiogram started to display on the screen (the time when the starting sound was produced from the electrocardiograph) after the end of the activation/preparation period. Thus, the timing of starting display of the electrocardiographic waveform and the timing of starting recording of the perspiration signals were synchronized with each other to make the elapsed time of measurement by both devices the same. The measurement time by the perspiration sensor (the recording time of the perspiration signals) was secured longer (by about several seconds) than the measurement time of the electrocardiograph, and the subject released the finger from the specimen unit (case) of the perspiration sensor after the end of the electrocardiogram measurement. It is noted that, in this experiment, a slight deviation (delay of several seconds) may occur from the start of recording of the perspiration signals until the subject places the finger on the specimen unit (case) of the perspiration sensor, but the waveform of the electrocardiogram and the waveform of the perspiration signals can be matched by the synchronization processing and adjustment of the measurement time described above.

Each subject was asked to perform the measurement described above one or more times per day, and the measurement was performed for three months. Although the total number of measurements differs depending on the subject, a total of 30 or more times of measurement was performed per subject.

Then, as exemplarily described above, using the analysis target data obtained by dividing the measurement period for 60 seconds into multiple frames having 4 seconds as one frame, regression analysis was performed using the above equations (1) to (4) to obtain the coefficients a and b and the constant α, and the T value was also calculated to evaluate the validity of the heart rate variability measurement of the present invention.

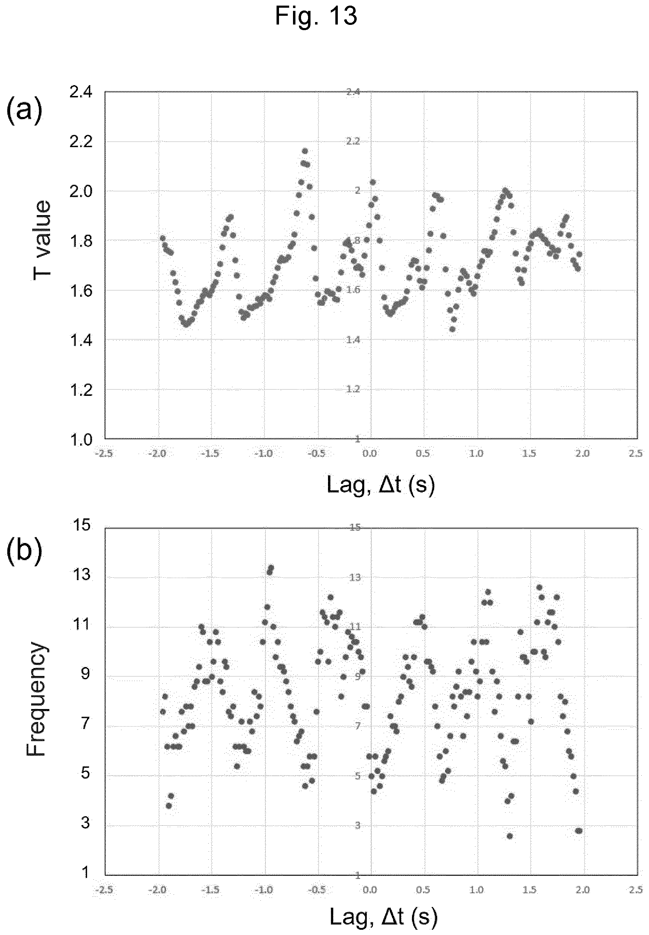

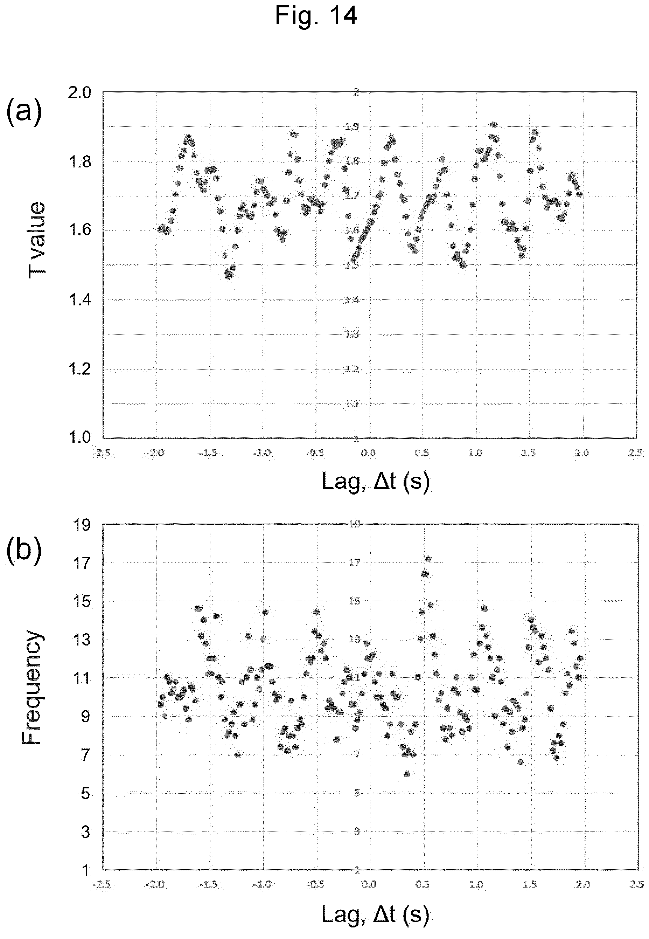

The results are shown in Figs. 13 and 14. In both Figs. 13 and 14, part (a) shows the T value with respect to the time lag Δt and part (b) shows the frequency of occurrence, Fig. 13 shows data acquired when the subject is at rest, and Fig. 14 shows data acquired when the subject is exercising (one round trip in climbing up and going down stairs for one floor). The constant α was set to 0.2 at which a high degree of relationship can be obtained from a large amount of data acquired in this example.

According to Fig. 13, a high T value of around 2 was obtained periodically for every time lag Δt of about 0.6 s, and thus it was demonstrated that heart rate variability can be measured with the heart rate variability measurement system of the present invention.

Also in Fig. 14, which is a measurement during exercise, it can be seen that a high T value is obtained periodically with for every time lag Δt of about 0.6 s. The peak T value is lower than that in Fig. 13 acquired when the subject is at rest, but this is considered to reflect a disturbance of the heart rate due to exercise.

It is noted that, in the present embodiment, a perspiration sensor employing a method of measuring a galvanic current flowing between thin wires is used. In this case, excellent responsiveness to perspiration and measurement accuracy, high accuracy with respect to a time lag Δt of 0.6 s, and stable measurement could be performed.

Industrial Applicability

As described above, the heart rate variability measurement system according to the present invention makes it possible to easily measure heart rate variability at a hand portion such as a finger with a small and lightweight measurement device. The daily measurement of the heart rate variability makes it possible to issue a warning to abnormality or an abnormal sign of the heart or the circulatory system, and can be effectively utilized for the management of the mental state and the biological rhythm.

Therefore, the heart rate variability measurement system and the heart rate variability measurement method of the present invention are considered to serve as a foundation for building a healthy society, and to be used widely in the industry field.

Reference Signs List

11    PERSPIRATION DETECTION UNIT
12    SIGNAL ARITHMETIC PROCESSING UNIT

| | |
|---|---|
| 21 | SPECIMEN UNIT |
| 21a | MICRODROPLET DETECTION UNIT |
| 22 | MEASUREMENT UNIT (PERSPIRATION SIGNAL OUTPUT UNIT) |
| 23 | FIRST THIN METAL WIRE |
| 24 | FIRST METAL ELECTRODE |
| 25 | SECOND THIN METAL WIRE |
| 26 | SECOND METAL ELECTRODE |
| 27 | WIRING |
| 28 | AMMETER |
| 29 | SIGNAL PATH |
| 31 | CASE |
| 32 | PROTECTIVE CAP |
| 41 | PROTECTIVE MESH |
| 51 | FINGER |
| 61 | SPACE |
| 71 | CALCULATION UNIT |
| 72 | INFORMATION STORAGE UNIT |
| 73 | HEART RATE VARIATION VALUE OUTPUT UNIT |
| 74 | PERSPIRATION SIGNAL, INPUT SIGNAL |
| 101 | HEART RATE VARIABILITY MEASUREMENT SYSTEM |
| 102 | SPECIMEN UNIT |
| 103 | SPECIMEN UNIT |
| 201 | SILICON CHIP (GALVANIC ARRAY) |
| 202 | FIRST THIN METAL WIRE (ALUMINUM) |
| 203 | SECOND THIN METAL WIRE (GOLD) |
| 204 | SCALE |

**Claims**

1.  A heart rate variability measurement system comprising:

    a perspiration detection unit, and a signal arithmetic processing unit, wherein
    the perspiration detection unit includes a microdroplet detection unit in which a first thin wire and a second thin wire are disposed in juxtaposition with each other on an insulating substrate, and a perspiration signal output unit that measures a change in an electrical characteristic between the first thin wire and the second thin wire and outputs the change as a perspiration signal,
    the signal arithmetic processing unit includes a calculation unit, an information storage unit, and a heart rate variation value output unit,

    while setting time t, a time lag $\Delta t$, a value CH(t) of the perspiration signal at the time t, a heart rate factor Y(t+$\Delta t$) indicating heart rate variability at time t+$\Delta t$, coefficients a and b, and a predetermined constant as $\alpha$, the calculation unit calculates following equations (1) and (2):

    $$Y(t+\Delta t)=a \cdot X(t)+b \quad \cdots (1)$$

    $$X(t)=CH(t)-CH(t-\alpha) \quad \cdots (2),$$

    the information storage unit stores at least the coefficient a, the coefficient b, and the constant $\alpha$, and
    the heart rate variation value output unit outputs the heart rate factor Y,
    where the coefficient a and the coefficient b are determined by obtaining a heart rate measurement heart rate factor Y' that is calculated from measurement of a heart rate value HB(t) at the time t, performed in advance simultaneously with the measurement of the perspiration signal by the heart rate variability measurement system, according to an equation (3):

    $$Y'(t+\Delta t)=HB(t+\Delta t)-HB(t+\Delta t-\alpha) \quad \cdots (3)$$

and performing regression analysis of an equation (4):

$$Y'(t+\Delta t)=a \cdot X(t)+b \quad \cdots \ (4).$$

2. The system as claimed in claim 1, wherein the perspiration detection unit includes a microdroplet detection unit in which a thin wire of first metal and a thin wire of second metal are disposed in juxtaposition with each other on an insulating substrate, the second metal being different from the first metal, and a perspiration signal output unit which measures a galvanic current flowing between the thin wire of the first metal and the thin wire of the second metal and outputs the galvanic current as a perspiration signal.

3. The system as claimed in claim 2, wherein the first metal is selected from a group consisting of gold, platinum, silver, titanium, an alloy thereof, and carbon.

4. The system as claimed in claim 2 or 3, wherein the second metal is selected from a group consisting of silver, copper, iron, zinc, nickel, cobalt, aluminum, tin, chromium, molybdenum, manganese, magnesium, and an alloy thereof.

5. The system as claimed in any one of claims 1 to 4, wherein the constant $\alpha$ is 0.2.

6. The system as claimed in any one of claims 2 to 5, comprising a plurality of at least one selected from the group consisting of the thin wire of the first metal and the thin wire of the second metal, wherein the thin wire of the first metal extends from a first side towards a second side that is opposite to the first side and the thin wire of the second metal extends from the second side towards the first side such that the thin wire of the first metal and the thin wire of the second metal run in parallel.

7. The system as claimed in any one of claims 2 to 6, wherein a spacing between the thin wire of the first metal and the thin wire of the second metal is 0.1 $\mu$m or more and 10 $\mu$m or less.

8. The system as claimed in any one of claims 1 to 7, wherein the perspiration detection unit and the signal arithmetic processing unit are placed at physically distant places.

9. The system as claimed in claim 8, wherein perspiration signal data output from the perspiration detection unit is transmitted to the signal arithmetic processing unit by communication.

10. The system as claimed in claim 8, wherein perspiration signal data output from the perspiration detection unit is transmitted to the signal arithmetic processing unit via an electronic medium.

11. A heart rate variability measurement method comprising:

acquiring a value CH(t) of a perspiration signal at time t using a perspiration detection device that includes a microdroplet detection unit in which a thin wire of first metal and a thin wire of second metal are disposed in juxtaposition with each other on an insulating substrate, the second metal being different from the first metal, and a perspiration signal output unit which measures a galvanic current flowing between the thin wire of the first metal and the thin wire of the second metal and outputs the galvanic current as a perspiration signal,
while setting a time lag $\Delta t$, a heart rate factor Y(t+$\Delta t$) indicating heart rate variability at time t+$\Delta t$, coefficients a and b, and a predetermined constant as $\alpha$, calculating following equations (1) and (2):

$$Y(t+\Delta t)=a \cdot X(t)+b \quad \cdots \ (1)$$

$$X(t)=CH \ (t)-CH(t-\alpha) \quad \cdots \ (2),$$

and
outputting the heart rate factor Y thus calculated,
where the coefficient a and the coefficient b are determined by obtaining a heart rate measurement heart rate factor Y' that is calculated from measurement of a heart rate value HB(t) at the time t, performed in advance simultaneously with the measurement of the perspiration signal by the perspiration detection device, according to an equation (3):

$$Y'(t+\Delta t)=HB(t+\Delta t)-HB(t+\Delta t-\alpha) \quad \cdot\cdot\cdot \quad (3)$$

and performing regression analysis of an equation (4):

$$Y'(t+\Delta t)=a\cdot X(t)+b \quad \cdot\cdot\cdot \quad (4).$$

**12.** The heart rate variability measurement method as claimed in claim 11, wherein the constant $\alpha$ is 0.2.

Fig. 1

101

11

12

# Fig. 2

Fig. 3

Humidity (Moisture)

CURRENT

ADSORPTION /
CONDENSATION

METAL B

WATER
DROPLET

METAL A

INSULATING
SUBSTRATE

Fig. 4

Fig. 5

Fig. 6

(a)

(b)

Fig. 7

Fig. 8

# Fig. 9

(a)

(b)

Fig. 10

(a)

(b)

Fig. 11

## Fig. 12

Push
start button

Start of Displaying of Electrocardiographic Waveform
(Starting sound is produced.)

End of Measurement
(silent)

Activation /
Preparation Period

Measurement Period (60 seconds)

Electro-
cardiograph

Time

Electrocardiograph is held while placing it near
heart.

Measurement Period (60 + several seconds)

Perspiration
sensor

Time

Finger is held while placing it on case of
sensor.

Start of Recordation
(synchronized with starting sound
produced from electrocardiograph)

End of Measurement
(Finger is released
from case after ending
of electrocardiogram
measurement )

# Fig. 13

(a)

(b)

# Fig. 14

<table>
<tr><td colspan="2" align="center">**INTERNATIONAL SEARCH REPORT**</td><td>International application No.<br><br>**PCT/JP2023/007447**</td></tr>
</table>

| | |
|---|---|
| **A. CLASSIFICATION OF SUBJECT MATTER** | |

*A61B 5/0245*(2006.01)i; *A61B 5/0533*(2021.01)i
FI:   A61B5/0245 100A; A61B5/0533

According to International Patent Classification (IPC) or to both national classification and IPC

**B.     FIELDS SEARCHED**

Minimum documentation searched (classification system followed by classification symbols)

A61B5/00-5/398

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); Scopus

**C.      DOCUMENTS CONSIDERED TO BE RELEVANT**

| Category* | Citation of document, with indication, where appropriate, of the relevant passages | Relevant to claim No. |
|---|---|---|
| A | WO 2018/150903 A1 (NATIONAL INSTITUTE FOR MATERIALS SCIENCE) 23 August 2018 (2018-08-23)<br>     entire text, all drawings | 1-12 |
| A | JP 2019-129965 A (LIFE KEA GIKEN KK) 08 August 2019 (2019-08-08)<br>     entire text, all drawings | 1-12 |
| P, A | WO 2022/196600 A1 (NATIONAL INSTITUTE FOR MATERIALS SCIENCE) 22 September 2022 (2022-09-22)<br>     entire text, all drawings | 1-12 |

| ☐ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **19 April 2023** | **09 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/007447**

| Patent document cited in search report | | | Publication date (day/month/year) | Patent family member(s) | | | Publication date (day/month/year) |
|---|---|---|---|---|---|---|---|
| WO | 2018/150903 | A1 | 23 August 2018 | US 2020/0053837 A1 entire text, all drawings EP 3584571 A1 | | | |
| JP | 2019-129965 | A | 08 August 2019 | (Family: none) | | | |
| WO | 2022/196600 | A1 | 22 September 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002345769 A

- WO 2016013544 A